# EUROPEAN PATENT APPLICATION

(11) **EP 2 937 059 A1**
(43) Date of publication of application: **28.10.2015**
(21) Application number: 15164644.5
(22) Date of filing: 22.04.2015
(51) Int. Cl.: A61C 7/00, A61C 7/12, A61C 7/14, A61C 7/20

(54) **ORTHODONTIC APPARATUS**

(30) Priority: 23.04.2014 US 201461983113 P
(71) Applicant: Tomy Incorporated, Futaba-gun Fukushima 979-1305 (JP)
(72) Inventor: ENDO, Kosei, Futaba-gun, Fukushima 9791305 (JP); HIKICHI, Masanobu, Futaba-gun, Fukushima 9791305 (JP); YUKISHITA, Yoshitaka, Futaba-gun, Fukushima 9791305 (JP)
(74) Representative: Grünecker Patent- und Rechtsanwälte PartG mbB

(57) **Abstract**

An orthodontic apparatus includes a composite material containing cellulose nanofibers and resin. The resin is thermoplastic resin or thermosetting resin.

## Description

### BACKGROUND

### TECHNICAL FIELD

The present invention relates to an orthodontic apparatus for correcting misaligned teeth and twisted teeth.

### RELATED ART

An orthodontic treatment is performed by mounting an archwire on a bracket or a tube mounted on a tooth and transmitting the restoring force of the archwire to the tooth. A bracket for use in such orthodontic treatment is known by the patent document 1 and the like.

A bracket currently available on the market is formed mainly of metal, ceramic or plastic.

The bracket includes, for example, an inexpensive and good molding-property bracket made of stainless steel such as SUS630, a titanium-made bracket for a patient having a metal allergy to a nickel or the like, and a bracket made of alumina ceramics or zirconia ceramics having good bio-compatibility and aesthetic appearance. Further, there is also known a polycarbonate-made bracket with good aesthetic appearance in which a stainless steel reinforcing material is embedded so as to form an archwire slot.

Also, the archwire includes, for example, a high-rigidity wire made of stainless steel or cobalt chrome alloy, a wire which is made of nickel titanium alloy or cobalt nickel alloy and thus has super-elasticity and is capable of effective treatment, a coated wire that a metal-made wire is coated with epoxy resin or fluorine resin for enhancing aesthetic appearance, and a resin-made wire which is formed of only resin.

From the viewpoint of cost, workability and easiness-to-handle, an orthodontic apparatus made of metal is mostly used.

[Patent Document 1] WO/2014/119089

Recently, there have been increasing patients who have medical checkup according to magnetic resonance imaging (MRI) using a magnetic resonator (MR) while they undergo the orthodontic treatment. In the orthodontic treatment, as described above, since the metal-made orthodontic apparatus is mostly used, the orthodontic apparatus must be removed before MRI photographing. Therefore, the patient must go to a clinic to remove the orthodontic apparatus by an orthodontist, and after MRI photographing, the patient must go to the clinic again to mount the orthodontic apparatus. This results in the delayed orthodontic treatment.

Also, in the orthodontic treatment using a bracket and a wire, for some of patients having a metal allergy to metal such as nickel and chrome, there must be used a bracket and a wire not containing nickel or chrome, from the early stage of the treatment to the completion of the treatment. In this case, since an orthodontic apparatus made of ceramic or titanium is used, the orthodontic treatment is expensive. Further, the orthodontic treatment can be executed using a plastic-made apparatus of a mouth-piece type which is called an aligner without using the metal-made apparatus at all. However, there are some cases that such apparatus cannot be used depending on the crowding of teeth.

Thus, a resin-made orthodontic apparatus having no influence on MRI photographing has been considered to be used as the orthodontic apparatus, such as the bracket and the archwire.

As the resin-made orthodontic apparatus, there have been developed a wire and a bracket, which are made of synthetic-resin containing reinforcing fibers such as glass fibers, PAN-based carbon fibers or pitch-based carbon fibers. However, when such synthetic-resin-made bracket is used in combination with a metal-made wire, a metal-made reinforcing member must be embedded in the bracket to reinforce a slot of the bracket. This is to prevent the resin-made bracket from being chipped or deformed by the metal-made wire.

Also, since resin is lower in material mechanical strength than metal, the diameter of the resin-made wire is larger than the diameter of the metal-made wire. Thus, the resin-made wire causes discomfort in an oral cavity and also can be used only in the specific time of the treatment.

Thus, there has been desired the development of a non-metal-made orthodontic apparatus which can be used through the orthodontic treatment period and having a mechanical strength equivalent to a metal-made orthodontic apparatus.

### SUMMARY

Exemplary embodiments of the invention provide an orthodontic apparatus which has good aesthetic appearance and need not be removed or mounted in the MRI photographing.

An orthodontic apparatus according to an exemplary embodiment of the invention comprises a composite material containing cellulose nanofibers and resin.

The resin may be thermoplastic resin or thermosetting resin.

The orthodontic apparatus of the exemplary embodiment of the invention is made of a composite material containing resin and plant-derived cellulose nanofibers as a reinforcing material, instead of glass fibers or carbon fibers. Thus, a bio-compatible orthodontic apparatus can be provided and, since the material is abundant as resources, its environmental load and manufacturing cost are small.

According to the exemplary embodiment of the invention, it is possible to provide an orthodontic apparatus which has good aesthetic appearance and need not be removed or mounted in the MRI photographing.

### BRIEF DESCRIPTION OF THE DRAWINGS

Fig. 1 is a perspective view showing a bracket to which the invention can be applied.
Figs. 2A and 2B are perspective views showing a self-ligating bracket to which the invention can be applied.
Figs. 3A and 3B are perspective views showing buccal tubes to which the invention can be applied.
Fig. 4 is a perspective view showing a wire to which the invention can be applied.
Figs. 5A to 5D are cross-sectional views showing wires to which the invention can be applied.
Fig. 6 is a perspective view showing a wire to which the invention can be applied.
Fig. 7 is a perspective view showing a lingual button to which the invention can be applied.
Fig. 8 is a perspective view showing a bracket to which the invention can be applied.
Figs. 9A to 9C are perspective views showing caps for a bracket to which the invention can be applied.
Figs. 10A to 10C are perspective views showing orthodontic apparatuses in each of which the cap of Fig. 9A, 9B or 9C is combined with the bracket of Fig. 8.

### DETAILED DESCRIPTION

Description is given below of an orthodontic apparatus according an embodiment of the invention with reference to the drawings.

Cellulose nanofibers for use in an orthodontic apparatus are materials derived from the cell wall of a plant. The cellulose nanofibers can be obtained by defibrating the cell wall down to a nano size. The cellulose nanofibers suitable for the orthodontic apparatus of the invention, for example, have a width of from 4 nm to 100 nm and a length of 5 µm or larger.

Resin for use in an orthodontic apparatus may be thermoplastic resin or thermosetting resin.

The thermoplastic resin can include, for example, polypropylene resin, polyethylene resin, polycarbonate resin, polyacetal resin, cycloolefin resin, polyether-ether-ketone resin, polysulfone resin, ABS resin, polyethylene-terephthalate resin, fluorine resin, polyvinyl resin, polyester resin, polyamide resin, acrylic resin, polyurethane resin, polybutylene terephtalate resin, and polyether-ketone ketone resin.

The thermosetting resin can include, for example, phenol resin, epoxy resin, urea resin, polyester resin, melamine resin, silicone resin, polyimide resin and polyurethane resin.

Here, these resins can be used singly or in combination of some of them.

Also, the orthodontic apparatus of the invention has a transparent or translucent appearance; however, in order to obtain a desired appearance, an arbitrary opaque color can be selected. For example, for matching to the color of teeth, additive such as colorant can also be added to the resin.

The orthodontic apparatus of the invention can be manufactured by mixing resin and cellulose nanofibers together to thereby produce a mixture and then forming the mixture into a desired shape. For forming the mixture into the desired shape, there can be used various methods, for example, an extrusion molding method, an injection molding method, a compression molding method, and a shaping method by cut machining. It is desirable that the cellulose nanofibers which are mixed with the resin have a hydrophobic property.

Further, for enhancement in the mechanical strength, a stretching step or a pressurizing and reinforcing step may be added, or the content rate of the cellulose nanofibers may be adjusted. Also, for the prevention for separation between the cellulose nanofibers and resin and the strength improvement, a special treatment may be subject to the surface of the cellulose nanofibers to increase areas of the interface between the cellulose nanofibers and the resin. Although a stretching ratio differs depending on the kinds of resin, the stretching ratio may be in the range of 1 to 10 times and, preferably, in the range of 2 to 5 times. Also, the content ratio of the cellulose nanofibers may be in the range of 0.5 to 85 wt% and, preferably, in the range of 2 to 70 wt%. Here, in order to facilitate inclusion into the resin, the average diameter of the cellulose nanofibers may preferably be in the range of 1 to 500 nm.

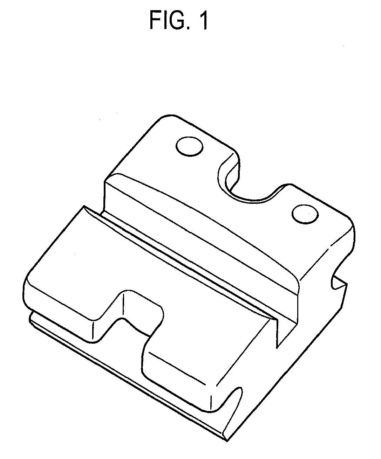

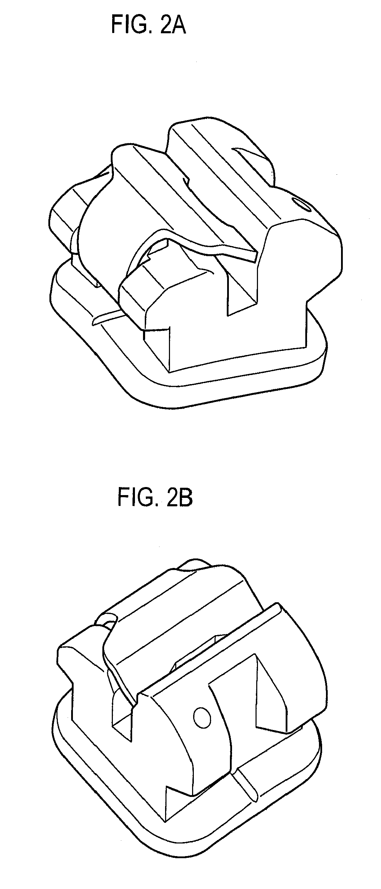

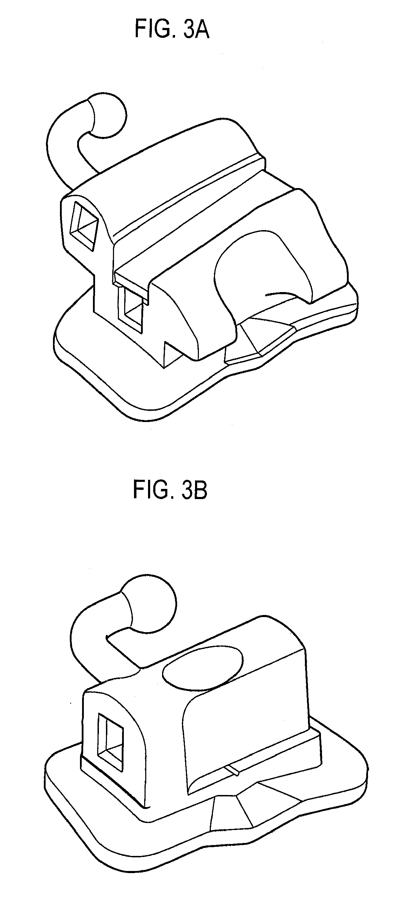

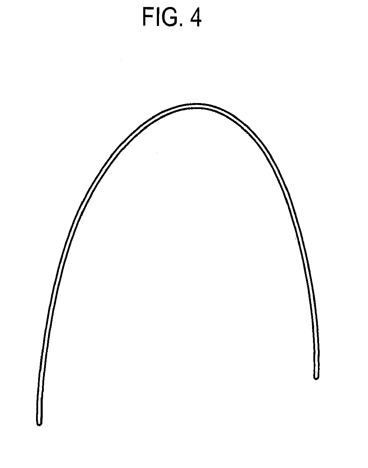

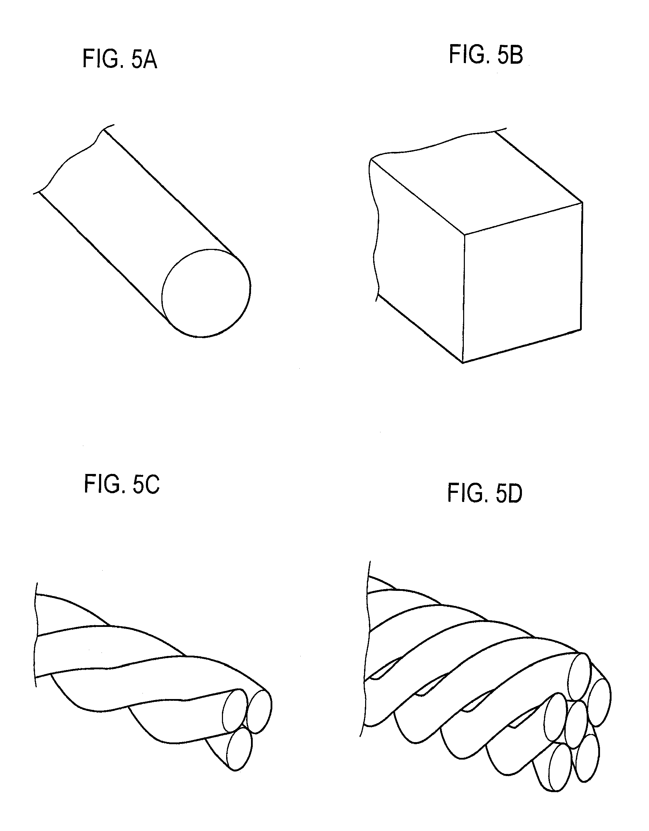

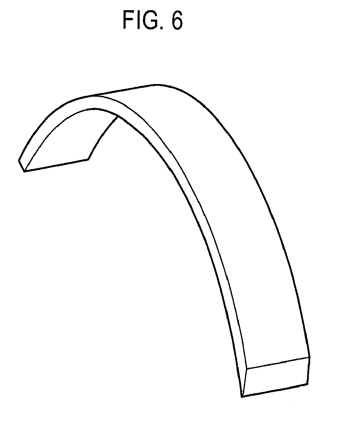

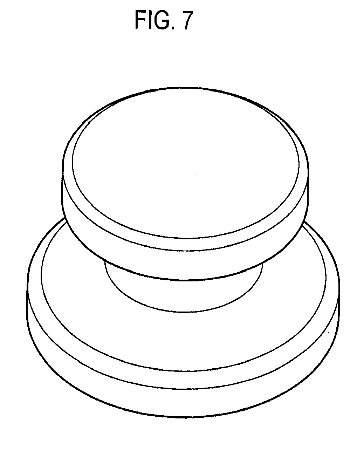

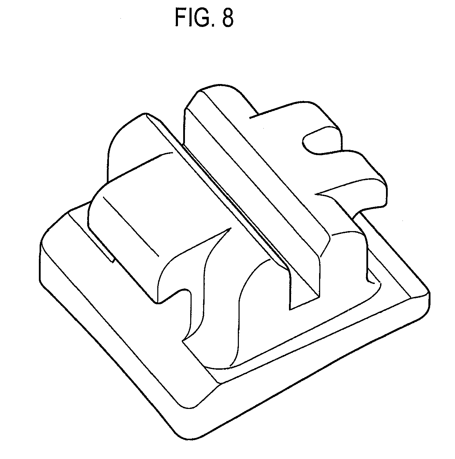

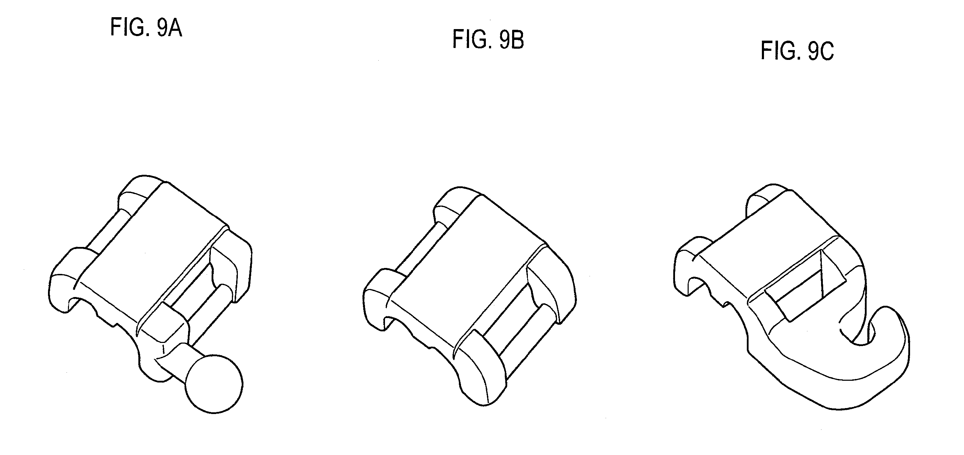

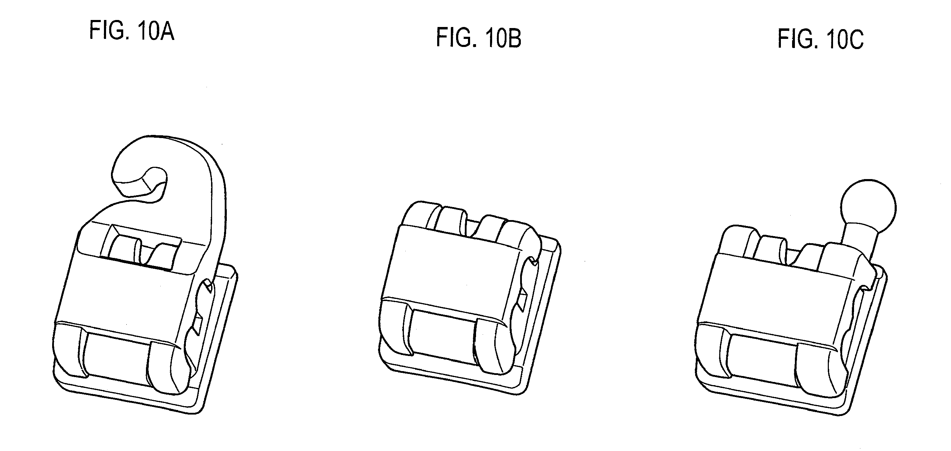

An orthodontic apparatus made of the above composite material includes all kinds of apparatuses for use in an orthodontic treatment, for example: an apparatus such as a bracket (Figs. 1, 2A, 2B, 8 and 10A to 10C), a cap (Figs. 9A to 9C), a buccal tube (Figs. 3A, 3B), a lingual button (Fig. 7) and a retainer, which are to be fixed to teeth using orthodontic adhesive; a wire (Figs. 4, 5A to 5D and 6) used in combination with the above fixed apparatuses (fixed sources); a band to be fitted into a tooth crown; and, a mouth piece type aligner. The bracket includes the self-ligation bracket (Figs. 2A, 2B). The wire may also be a wire the cross-section of which has a circular shape (Fig. 5A) or a rectangular shape (Fig. 5B). The wire may also be formed by twisting together three wires (Fig. 5C), by twisting together six wires (Fig. 5D). Or, a belt-shaped wire may also be used (Fig. 6).

The tensile strength of an apparatus serving as a fixed source, for example, a bracket and a buccal tube may be preferably 30 MPa or larger, more preferably, 40 MPa or larger. The hardness of the apparatus serving as the fixed source may preferably be HRR 40 (Rockwell hardness) or larger.

The tensile strength of an apparatus such as a wire to be used while changing its shape within an oral cavity may be preferably 50 MPa or larger, more preferably, 70 MPa or larger. Also, the maximum load in a bending test may be preferably 0.8 N or larger, more preferably, 1.5 N or larger.

Also, the amount of water absorption of the orthodontic apparatus of the invention may be preferably 50 µg/mm³ or less, more preferably, 30 µg/mm³ or less. The dissolving amount of the orthodontic apparatus of the invention may be preferably 5 µg/mm³ or less, more preferably, 1.6 µg/mm³ or less.

## Claims

1. An orthodontic apparatus comprising a composite material containing cellulose nanofibers and resin.

2. The orthodontic apparatus according to Claim 1, wherein the resin is thermoplastic resin or thermosetting resin.
